# EUROPEAN PATENT APPLICATION

(11) **EP 4 850 526 A1**
(43) Date of publication of application: **09.12.2026**
(21) Application number: 24906606.9
(22) Date of filing: 07.10.2024
(51) Int. Cl.: A01K 27/00, A01K 29/00, G16H 40/67

(54) **HARNESS FOR PETS**

(30) Priority: 19.12.2023 ES 202332248 U
(71) Applicant: Quantum Sync, SL., 29005 Malaga (ES)
(72) Inventor: MUNERA SILVA, Fernayn, 29005 MALAGA (ES)
(74) Representative: González López-Menchero, Álvaro Luis
(86) International application number: PCT/ES2024/070610
(87) International publication number: WO 2025/133419

(57) **Abstract**

The present invention relates to a smart harness for pets, which has different AI-managed functionalities and is formed by a strong casing comprising a microprocessor assisted by its corresponding electric battery, a communications module with its corresponding SIM card, a GPS positioning module, an internal memory, wireless communication modules, a video camera, a microphone, at least one speaker and means for monitoring and controlling the animal's health. All the data collected by said AI-managed elements are transmitted to an externally controlled mobile device, from which the user tracks the animal.

## Description

### TECHNICAL FIELD

The present invention relates to a smart harness, chest harness, or collar for pets, such as dogs and cats, without excluding other animals that require similar features. In particular, the invention focuses on the integration of Artificial Intelligence (AI) technology into the harness to provide advanced assistance, monitoring, and safety features for both pets and the people who interact with them.

### BACKGROUND OF THE INVENTION

In the field of pets, devices that enable GPS tracking or basic communication with the animal are well known. However, these devices typically offer limited features and do not fully leverage the possibilities offered by Artificial Intelligence to improve the interaction, care, and safety of pets and their owners.

There is a growing need for devices that can assist people with conditions such as Alzheimer's, as well as monitor the cognitive health of pets, including the early detection of senile dementia in dogs.

### DESCRIPTION OF THE INVENTION

The proposed smart pet harness integrates Artificial Intelligence technology to provide a series of advanced features, described below:
1. AI-powered GPS tracking
   The harness includes an AI-assisted GPS tracking module (10) that tracks the animal's location in real time and learns its movement patterns. The AI analyzes these patterns and, if it detects that the animal enters dangerous areas or exhibits abnormal behavior, sends automatic alerts to the owner's control device.
2. Remote communication with the dog
   Using biometric and behavioral sensors, the AI detects signs of anxiety or stress in the dog. In response, it automatically activates remote communication, allowing the owner to calm the dog using voice commands through the speaker (16) built into the harness.
3. Visualization of the dog's surroundings
   The harness incorporates a 360° video camera (14) that, in combination with AI, analyzes the captured images in real time to detect hazards in the dog's environment. The AI can identify dangerous objects or nearby animals and alert the owner to potential risks.
4. Generation of alarms based on preset parameters
   The AI monitors the dog's movements within a set perimeter and learns its habits. If the dog attempts to leave the safe area or shows signs of stress, the system sends alerts to the owner.
5. Playing soothing music
   Based on the dog's bio metric data, the AI detects moments of anxiety or nervousness and automatically plays soothing music through the speaker (16) to help calm the animal.
6. Pet identification in case of loss
   The AI analyzes the dog's surroundings and behavior and, if necessary, generates an identification message on the harness's display (20), providing useful information to anyone who finds the dog.
7. Adjustable LED lighting
   The harness features LED lights (21) whose intensity and color are automatically adjusted by the AI based on visibility in the environment, optimizing lighting at night or in dark areas to improve the dog's safety.
8. Animal health monitoring
   The AI constantly analyzes the dog's biometric data, such as heart rate (measured by the heart rate monitor (22)), temperature (measured by the temperature sensor (24)), and physical activity (measured by the accelerometer (25) and gyroscope (26)), to detect potential health issues, including symptoms of senile dementia. It sends alerts to the owner for early intervention.
9. Detection of aggressive dogs and avoidance response
   Using the camera and additional sensors, the AI detects the presence of other dogs and analyzes their behavior. If it identifies aggressive behavior, it activates an automatic deterrent system, such as emitting ultrasonic sounds through the ultrasonic emitter (27), to protect the dog.
10. Bark control The AI learns the dog's normal barking patterns and distinguishes between normal barking and barking that could indicate anxiety or danger. The system automatically adjusts bark control based on the context.
11. Remote Harness Control via Mobile Phone
   The AI provides recommendations to the owner on when to remotely control the harness, based on continuous analysis of the dog's behavior and its environment.
12. Harness battery optimization
   The AI optimizes battery usage (6) by temporarily disabling non-essential functions to save energy. Additionally, it issues alerts when the battery needs to be recharged.
13. Unlocking with a security code
   The AI monitors the dog's surroundings and situation, suggesting when it is safe to unlock the harness if it detects that the dog is in an emergency situation.
14. Water resistance and durability
   The AI adjusts the harness's performance in adverse conditions, such as underwater, and automatically activates protective measures when it detects risks, such as accidental cutting or prolonged submersion.
15. AI-powered parasite treatment
   AI analyzes the dog's environment and conditions and activates the pest control release device (33) at specific times, optimizing its effectiveness based on the location and situation.
16. Collaboration with drones for search and rescue
   The harness enables direct communication with drones during search operations, transmitting in real time the location of the dog or the person who needs to be found, such as someone with Alzheimer's who has become disoriented.
17. Screen for emergency messages and pet data
   AI generates messages on the harness's screen (20) if the dog is in danger or needs help. These messages may include useful information about the dog and its health status, helping bystanders take action.

### NEW FEATURES RELATED TO AI AND ALZHEIMER'S/SENILE DEMENTIA

### A) Assisting People with Alzheimer's Using AI: The AI learns the movements and behavioral patterns of the person with Alzheimer's.

If it detects disorientation or straying from safe areas, the harness sends automatic alerts to the caregiver and can activate the dog to guide the person back to a safe place using auditory and visual signals.

### B) Monitoring Dogs with Senile Dementia

AI continuously monitors the dog's behavioral data to detect early signs of senile dementia. When signs of cognitive decline are detected, the harness activates soothing sounds and vibrations to reduce the dog's stress and sends alerts to the owner so they can take action.

### C) AI-Powered Radiation Protection

The harness includes radiation sensors (32) that detect dangerous levels in the environment.

The AI assesses the risk and adjusts the harness's functions to protect the dog, issuing automatic alerts to the owner about the need to remove the animal from the affected area.

### D) AI-Assisted Dog Response

In an emergency, the AI coordinates the dog to assist the person with Alzheimer's or in a dangerous situation. The dog can activate visual or auditory alerts and guide the person back to a safe area.

The harness according to the present invention is described in claim 1, and the dependent claims include additional features that are optional.

To this end, and based on the conventional structure of any pet harness, chest harness, or collar, the harness of the invention focuses its features on the fact that it consists of a housing integrated into the harness itself, within which a control circuit is established that includes a microprocessor, powered by its corresponding rechargeable battery via a connection port, and/or via a wireless charging module; this circuit includes a communications module with its corresponding 4G/5G SIM card, as well as a GPS location module, internal memory, and Wi-Fi/Bluetooth wireless communication modules.

The harness also includes a camera, a microphone, and a speaker, which allow you to see where the animal is located and communicate with it, as well as with anyone who may have come across the animal if it gets lost.

In this way, the harness can be controlled via an app installed on a smartphone, smartwatch, or similar device, or via a remote control designed for this purpose.

The control software allows users to set a control perimeter based on the maximum distance the animal can travel from the phone, remote, or device in question, so that once that distance is exceeded, an alarm signal is triggered on the mobile phone or control device.

The harness's speaker(s) can also be used to play soothing music for the animal or, as mentioned earlier, to communicate with it.

The harness can also incorporate a small screen through which messages can be sent in case of loss, or the pet's information can be entered.

According to another feature of the invention, the harness may incorporate LED lights, adjustable via the associated mobile phone app or control device in question.

In addition, the harness is designed to include means for monitoring the animal's health, such as a heart rate monitor, glucose meter, temperature sensor, accelerometer, and gyroscope (the latter two for calculating steps and calories burned).

Based on these parameters, it is possible to monitor the pet's health and generate reports accessible via the control device (smartphone/smartwatch, Tablet, etc.).

As for the harness clasp itself, it will be equipped with an electronic lock that can only be opened by entering a code via the control device.

The harness will be waterproof, so it can be submerged in water, and is made from durable materials that make it resistant to cuts and wear.

Optionally, the harness may incorporate an ultrasonic emitter, so that once the microphone detects the barking of a potentially dangerous dog, the harness itself will emit an ultrasonic signal to scare the dog away without causing it harm.

This feature can also be used to control the pet's own barking.

As for the harness's speakers, they can be used for training the pet.

Finally, it should be noted that the collar, harness, or chest strap can incorporate a receptacle for antiparasitic products in any format available on the market.

According to a preferred embodiment, the video camera is a 360° camera.

Furthermore, the pet harness according to the present invention may also comprise a thermal camera, a fingerprint sensor, at least one solar panel connected to the battery, a gas sensor, a radiation shield, and/or a flea repellent element.

Finally, the current possibilities offered by Artificial Intelligence (AI) technology are countless, and the following lists the various functionalities of the pet harness that are compatible with this technology.

### AI-powered GPS tracking:

AI tracks the dog in real time and learns its movement patterns. If it detects that the dog enters a dangerous area or exhibits abnormal behavior, it sends automatic alerts to the owner.

Remote communication with the dog:
The AI can detect signs of anxiety or stress in the dog and automatically activates remote communication with the owner so they can calm the dog using voice commands.

### Visualization of the dog's surroundings:

Using AI, the collar analyzes images captured in real time, detecting hazards in the dog's environment and alerting the owner to potential risks, such as dangerous objects or nearby animals.

### Alarm generation in predefined perimeters:

The AI not only monitors the dog's movements within the established perimeter, but also learns its habits and issues alerts if the dog attempts to leave the safe area or shows signs of stress.

### Playing soothing music:

The AI detects moments of anxiety or nervousness in the dog based on its biometric data, automatically activating soothing music to help calm the animal.

### Pet identification in case of loss:

The AI analyzes the dog's surroundings and behavior and, if necessary, generates an identification message on the collar that provides useful information to anyone who finds the dog.

### Adjustable LED lighting:

The AI adjusts the collar's LED lights based on visibility in the environment, optimizing lighting at night or in dark areas to improve the dog's safety.

### Pet health monitoring:

AI constantly analyzes the dog's biometric data, such as heart rate, temperature, and physical activity, to detect potential health issues, including symptoms of senile dementia. It sends alerts to the owner for early intervention.

### Detection of aggressive dogs and avoidance response:

AI detects the presence of other dogs and analyzes their behavior. If it identifies aggressive behavior, it activates an automatic deterrent system using ultrasonic sounds to protect the dog.

### Bark control:

The AI learns the dog's normal barking patterns and distinguishes between normal barking and barking that could indicate anxiety or danger. The collar automatically adjusts bark control based on the context.

### Remote control of the collar via mobile phone:

The AI provides recommendations to the owner on when to remotely control the collar, based on continuous analysis of the dog's behavior and its environment.

### Charging the collar:

AI optimizes battery usage by temporarily disabling non-essential features to save power. It also sends alerts when the battery needs recharging.

### Unlocking with a security code:

The AI monitors the dog's surroundings and situation, suggesting when it is safe to unlock the collar if it detects that the dog is in an emergency situation.

### Waterproofing and durability:

The AI adjusts the collar's performance in adverse conditions (such as underwater) and automatically activates protective measures when it detects risks, such as accidental cutting or prolonged immersion.

### AI-powered parasite treatment:

The AI analyzes the dog's environment and condition, and activates the parasite treatment dispenser at specific times, optimizing its effectiveness based on the location and situation.

### Collaboration with drones for search operations:

AI enables direct communication with drones during search operations, transmitting in real time the location of the dog or the person who needs to be found, such as someone with Alzheimer's who has become disoriented.

### Screen for emergency messages and pet data:

AI generates messages on the collar's screen if the dog is in danger or needs help. These messages can include useful information about the dog and its health status, helping bystanders take action.

Additionally, the use of Artificial Intelligence unlocks new functionalities related to this technology and Alzheimer's/Senile Dementia

### A) Assisting People with Alzheimer's Using AI:

The AI learns the movements and behavioral patterns of the person with Alzheimer's. If it detects disorientation or straying from safe areas, the harness sends automatic alerts to the caregiver and can activate the dog to guide the person back to a safe place**.**

### B) Monitoring Dogs with Senile Dementia:

AI continuously monitors the dog's behavioral data to detect early signs of senile dementia. When signs of cognitive decline are detected, the harness activates soothing sounds and vibrations to reduce the dog's stress and sends alerts to the owner so they can take action.

### C) AI-Powered Radiation Protection:

The collar includes sensors that detect dangerous levels of radiation in the environment.

AI assesses the risk and adjusts the collar's functions to protect the dog, by sending automatic alerts to the owner regarding the need to remove the animal from the affected area.

### D) AI-Assisted Dog Response:

In an emergency, the AI coordinates the dog to assist the person with Alzheimer's or in a dangerous situation. The dog can activate visual or auditory alerts and guide the person back to a safe area.

In conclusion, it can be said that this pet harness integrates AI into all 17 functions of the smart collar and adds significant improvements in assisting people with Alzheimer's and monitoring dogs with senile dementia. Additionally, radiation protection is now built-in and automatically managed by AI, ensuring early intervention in risky situations. Each function is designed to maximize the safety and well-being of both animals and the people who interact with them.

### BRIEF DESCRIPTION OF THE DRAWINGS

To supplement the description provided below and to aid in a better understanding of the invention's features, in accordance with a preferred embodiment of the invention, a set of drawings is included as an integral part of this description. These drawings illustrate, by way of example and not limitation, the following:

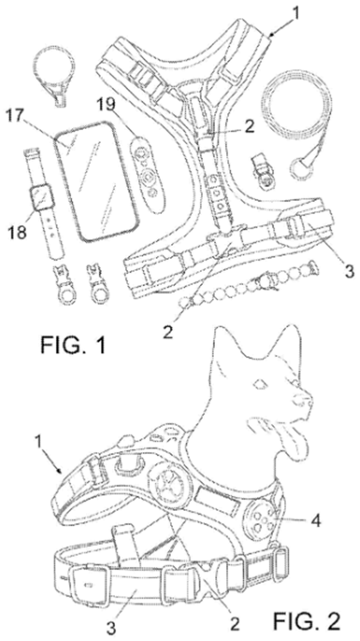

Figure 1.- Shows a top view of a pet harness made in accordance with the subject matter of the present invention.
Figure 2.- Shows a perspective view of the harness of Figure 1.

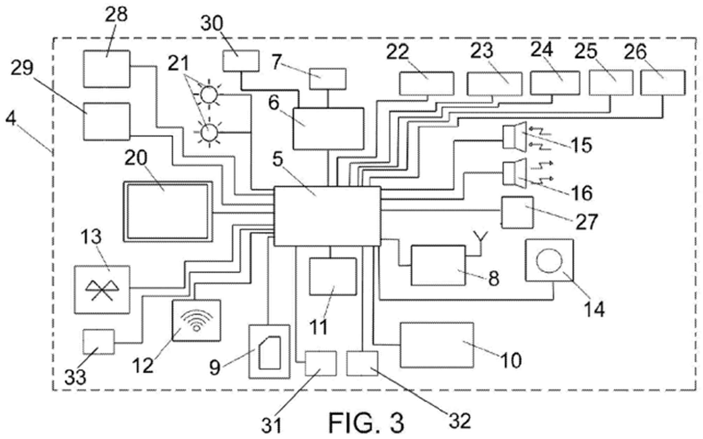

Figure 3.- Finally, shows a schematic block diagram of the harness's internal electronics.

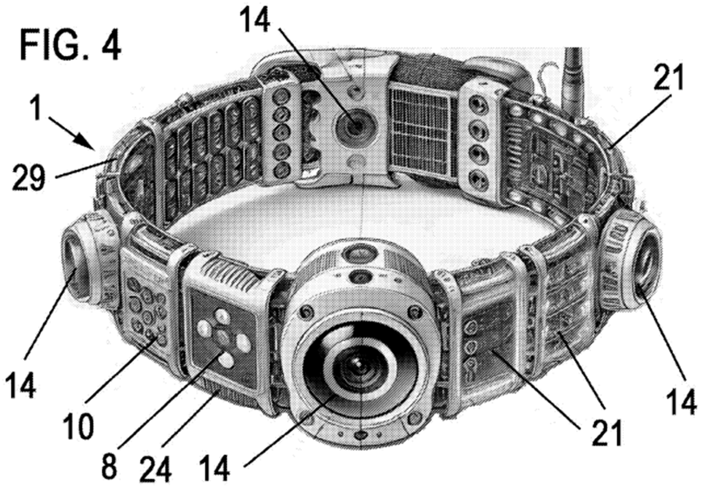

Figure 4.- Shows a perspective view of the harness according to the present invention, in accordance with one embodiment.
Figure 5.- Shows a perspective view of the harness according to the present invention, in which the encapsulated gas sensor is shown.
Figure 6.- Shows a perspective view of the harness according to the present invention, in which the life jacket is shown.

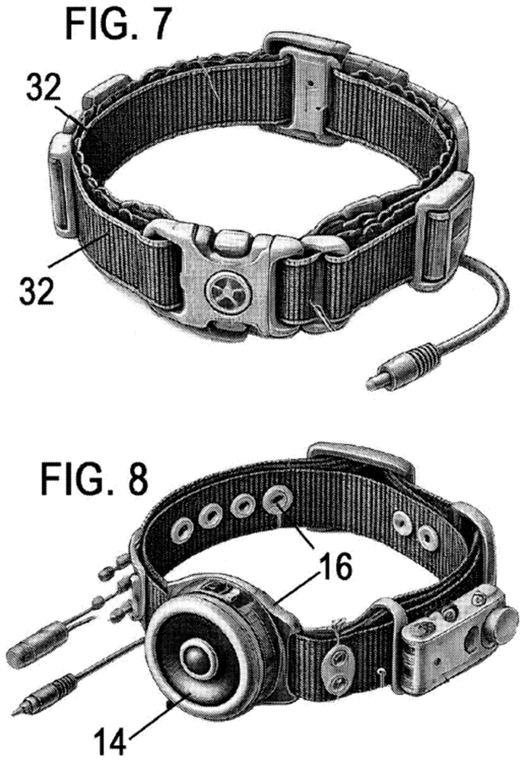

Figure 7.- Shows a perspective view of the harness according to the present invention, in which the radiation shield is shown.
Figure 8.- Shows a perspective view of the harness according to the present invention, in which the aids for visually impaired persons are shown.

### PREFERRED EMBODIMENT OF THE INVENTION

In view of the figures described, it can be seen that the invention is based on the conventional structure of a harness, chest harness, or collar for pets; in the chosen example, a harness (1), with its corresponding fastening (2) and adjustment (3) mechanisms.

According to the invention, the harness is designed to include a durable housing (4) that is integrated into the harness itself, within which a control circuit is housed that includes a microprocessor (5), powered by a corresponding rechargeable battery (6) via a connection port (7) and/or via a wireless charging module.

The circuit includes a communications module (8), with its corresponding 4G/5G SIM card (9), as well as a GPS location module (10), an internal memory (11), and Wi-Fi/Bluetooth wireless communication modules (12-13).

This communications module (8) is preferably integrated into the harness (1) and enables long-distance communication, improving the ability to track and communicate with the animal over large areas.

For example, this communications module (8) enables the harness (1) to communicate with drones for search and rescue operations.

In addition, the circuit includes a video camera (14), a microphone (15), and at least one speaker (16).

This camera (14) may be a 360° camera, which is preferably located on the top of the harness (1). This 360° camera (14) provides a complete view of the animal's surroundings, enabling real-time video recording and transmission, which is ideal for monitoring the animal's environment and is particularly useful in surveillance or exploration situations.

The 360° camera (14) and the communications module (8) can convert the images into audio data, allowing visually impaired people to understand the environment during the animal's walk.

Meanwhile, the microphone (15) and speaker (16) can be located on one side of the harness (1), and their function is to enable two-way communication between the owner and the animal, allowing the owner to speak to the animal and hear its surroundings, thereby improving communication and control.

This speaker (16) can also be used for features designed for visually impaired users, providing audible signals to improve the animal's interaction with visually impaired owners. This speaker (16) could be supplemented with a vibrator to provide vibrations for interacting with visually impaired owners.

The microprocessor (5) will be supported by programming software that allows it to be controlled via an external control device, such as a mobile phone (17), smartwatch (18), or similar device, or via a remote control (19) designed for this purpose.

As previously mentioned regarding GPS, the software will allow for the establishment of a control perimeter based on a preprogrammed maximum distance radius, such that once that distance is exceeded, an alarm signal is triggered on the control device.

This GPS (10) is preferably high-precision and can be integrated into the back of the harness. In addition to the above function, it can also provide real-time tracking and location of the animal, allowing the owner to track the animal's location at all times via a mobile app.

Optionally, the harness may additionally incorporate a display (20) through which messages can be sent in case of loss, or the pet's information can be entered.

This display (20) can be a multicolor LED display, preferably located around the harness (1). Its function is to provide visual signaling for greater visibility and communication with the animal, improving the animal's visibility in the dark and allowing for the display of customized visual messages as needed.

Through the aforementioned software, the color of a series of LEDs (21) distributed across the surface of the harness can also be adjusted, making the pet more visible when walking at night.

These LEDs (21) also help improve the animal's interaction with owners who are hearing impaired.

These LEDs (21) can be replaced or supplemented with strobe lights, located around the harness (1), which provide visual signals in emergency situations, increasing the animal's visibility and safety in dangerous situations.

These LEDs (21) include means for adjusting their brightness via the external control device.

According to another feature of the invention, the harness is designed to include means for monitoring and controlling the animal's health, namely biometric sensors, to generate health reports and prevent diseases.

These biometric sensors or monitoring and control means may be integrated into the harness (1), allowing for the monitoring of the animal's vital signs, enabling the owner to maintain real-time control over the animal's health.

Notable among these means are a heart rate monitor (22), a glucose meter (23), a temperature sensor (24), an accelerometer (25), and a gyroscope (26), the latter two used to calculate steps and calories burned.

Optionally, the harness may incorporate an ultrasonic emitter (27) to control the dog's barking and/or scare away other dogs that may approach.

In addition, the harness's fastening mechanisms (2) will be equipped with an electronic lock that can only be opened by entering a code via the control device; the harness includes at least one receptacle for antiparasitic products in any format available on the market.

The harness (1) according to the present invention may also comprise a thermal camera (28), which is preferably integrated alongside the camera (14) on the upper part of the harness (1).

This thermal camera (28) detects heat, which can be useful in search and rescue operations, helping to locate animals or people in low-visibility conditions or in the dark.

The harness (1) according to the present invention may also comprise a fingerprint sensor (29), which is preferably located on the front of the harness (1).

This sensor (29) enables authentication of the owner or authorized user to prevent unauthorized use, ensuring that only the owner or authorized user can activate or deactivate the functions of the harness (1).

The harness (1) according to the present invention may also comprise one or more solar panels (30) preferably located on the surface of the harness (1).

Their function is to continuously recharge the battery (6) of the harness (1) during the day, increasing the range of the harness (1) and making it more efficient and environmentally friendly.

The harness (1) according to the present invention may also comprise a gas sensor (31) integrated into the harness (1), which allows for the detection of hazardous gases in the environment, improving safety in areas at risk of toxic gases. The sensor (31) is encapsulated in durable materials, ensuring its protection against adverse conditions.

The harness (1) according to the present invention may also comprise a radiation shield (32) integrated into the harness (1), which provides protection against harmful radiation, thereby increasing the animal's safety in environments with radiation.

The harness (1) according to the present invention also comprises a rechargeable flea repellent element (33), which is preferably integrated into the harness (1), providing an effective and sustainable solution against fleas.

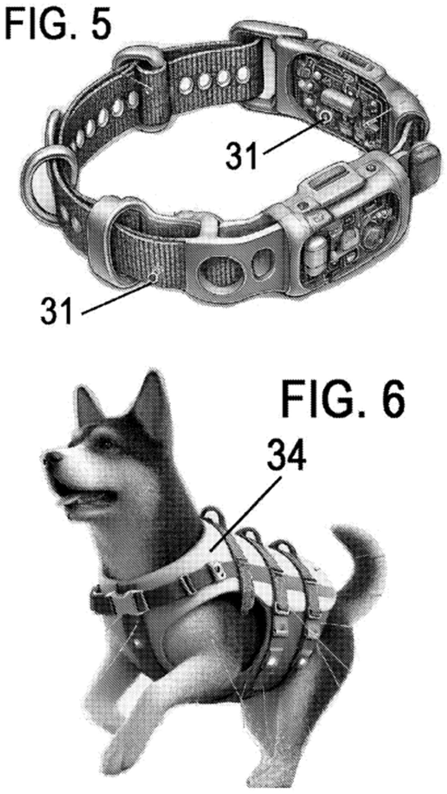

In addition, a life jacket (34) may be used in conjunction with the harness (1) according to the present invention, which is placed over the animal, as shown in Figure 6.

This life jacket (34) provides additional safety during aquatic activities, improving the animal's safety in the water, ensuring its buoyancy, and facilitating rescue in case of an emergency.

The harness (1) is constructed from a sturdy shell (4) integrated into it, inside which is a control circuit that includes:
- Microprocessor (5): Programmed with AI algorithms to process sensor data and control advanced features.
- Rechargeable battery (6): Rechargeable via a connection port (7) and/or a wireless charging module.
- Communications module (8): With a 4G/5G SIM card (9) for long-distance communication and connection to external devices.
- GPS tracking module (10): For real-time tracking of the animal.
- Internal memory (11): For storing data and programs.
- Wi-Fi and/or Bluetooth wireless communication modules (12-13): For communication with nearby devices.
- 360° video camera (14) and optional thermal camera (28): For capturing images of the surroundings.
- Microphone (15) and speaker (16): For two-way communication and sound output.
- Display (20): To show messages and relevant data.
- Biometric sensors: Including heart rate monitor (22), glucose meter (23), temperature sensor (24), accelerometer (25), and gyroscope (26).
- Ultrasonic emitter (27): To control barking and deter other animals.
- Fingerprint sensor (29): For user authentication.
- Solar panels (30): For continuous battery charging.
- Gas (31) and radiation (32) sensors: For detecting environmental hazards.
- Rechargeable flea repellent (33): For parasite treatment.
- LED lights (21): Distributed across the surface of the harness, controlled by Al.

## Claims

1. Smart pet harness, which, based on the conventional structure of a pet harness, chest harness, or collar, with its corresponding fastening (2) and adjustment (3) mechanisms, is **characterized by** including a durable housing (4) integrated therein, within which a control circuit is established comprising:
- A microprocessor (5) programmed with Artificial Intelligence (AI) algorithms to analyze, in real time, data from sensors integrated into the harness (1) and manage the harness's advanced functionalities;
- An electric battery (6), rechargeable via a connection port (7) and/or via a wireless charging module;
- A communications module (8), with its corresponding 4G/5G SIM card (9), for long-distance communication and data transmission;
- An AI-assisted GPS tracking module (10) for advanced animal tracking;
- An internal memory (11);
- Wi-Fi and/or Bluetooth wireless communication modules (12-13);
- A video camera (14), preferably a 360° camera, for visual analysis of the environment in combination with AI;
- A microphone (15) and at least one speaker (16), for two-way communication and the playback of AI-controlled sounds;
- Biometric sensors for monitoring and controlling the animal's health, including a heart rate monitor (22), a glucose meter (23), a temperature sensor (24), an accelerometer (25), and a gyroscope (26);
- A display (20) and a plurality of LEDs (21) distributed across its surface, controlled by the AI to adjust their brightness and provide visual signals;
- An electronic lock for the fasteners (2), managed by the AI to provide additional security;
- An encapsulated gas sensor (31) and a radiation shield (32), where the AI assesses environmental risks and adjusts the harness's functions accordingly;
- An external control device using control software, which allows interaction with and configuration of the functionalities managed by the AI.

2. A pet harness according to claim 1, **characterized in that** it also comprises a life jacket (34).

3. A pet harness according to claim 1, **characterized in that** the means for monitoring and controlling the animal's health consist of a heart rate monitor (22), a glucose meter (23), a temperature sensor (24), an accelerometer (25), and a gyroscope (26).

4. A pet harness according to claim 1, **characterized in that** it incorporates an ultrasonic emitter (27).

5. Pet harness according to claim 1, **characterized in that** the control means comprise a mobile phone (17), a smartwatch (18), and/or a remote control (19).

6. Pet harness according to claim 1, **characterized in that** the video camera (14) is a 360° camera.

7. A pet harness according to claims 1 and 6, wherein the 360° camera (14) and the communications module (8) comprise means for converting the images into audio data emitted by the speaker (16).

8. A pet harness according to any of the preceding claims, **characterized in that** it also comprises a thermal camera (28).

9. A pet harness according to any of the preceding claims, **characterized in that** it also comprises a fingerprint sensor (29).

10. A pet harness according to claim 1, **characterized in that** it also comprises at least one solar panel (30) connected to the battery (6).

11. A pet harness according to any of the preceding claims, **characterized in that** it also comprises a flea repellent element (33).
